# EUROPEAN PATENT APPLICATION

(11) **EP 4 843 474 A1**
(43) Date of publication of application: **25.11.2026**
(21) Application number: 25741972.1
(22) Date of filing: 16.01.2025
(51) Int. Cl.: A61B 1/00, A61B 1/005, A61M 25/00, G02B 23/24

(54) **FLEXIBLE TUBE FOR INSERTION APPARATUS, INSERTION APPARATUS, AND METHOD FOR MANUFACTURING FLEXIBLE TUBE FOR INSERTION APPARATUS**

(30) Priority: 17.01.2024 US 202463621631 P
(71) Applicant: Olympus Medical Systems Corp., Tokyo 192-8507 (JP)
(72) Inventor: INOUE Masato, Hachioji-shi, Tokyo 192-8507 (JP); SUZUKI Hisanobu, Hachioji-shi, Tokyo 192-8507 (JP)
(74) Representative: Gunzelmann, Rainer
(86) International application number: PCT/JP2025/001074
(87) International publication number: WO 2025/154747

(57) **Abstract**

A universal cord 1 of an endoscope 9 includes an elongated cylinder 10 having a first end 10A and a second end 10B, and including a spiral tube 20 formed by spirally winding a strip-shaped plate, and a tube 30 having flexibility and provided on an outer circumferential surface of the spiral tube 20; two substantially cylindrical ferrules including a first ferrule 41 fixed to the first end 10A, and a second ferrule 42 fixed to the second end 10B; and a wire 50 having a third end 50A and a fourth end 50B, the third end 50A being fixed to the first ferrule 41, the fourth end 50B being fixed to the second ferrule 42, the wire being inserted in through the cylinder 10 and having an elongation rate smaller than an elongation rate of the cylinder 10.

## Description

### BACKGROUND OF THE INVENTION

### 1. Field of the Invention

Embodiments of the present disclosure relate to a bendable tube for an insertion instrument, an insertion instrument, and a method for manufacturing the bendable tube for the insertion instrument.

### 2. Description of Related Art

A universal cord of an endoscope, which is a bendable tube for an insertion instrument, is a connecting cord between an operation portion of an endoscope and a connector to be connected to external instruments (for example, a video processor, a light source apparatus).

Japanese Patent Laid-Open Publication No. H11-332818 discloses a universal cord configured of a laminated structure obtained by sequentially layering a flex which is a spiral tube formed by spirally winding a ribbon-like thin plate, a braided tube which is a mesh tube woven from fine metal wires, synthetic fibers, or the like, and an outer sheath (tube) formed from resin.

### PRIOR ART DOCUMENT

### PATENT DOCUMENT

Patent Document 1: Japanese Patent Laid-Open Publication No. H11-332818

Universal cords (bendable tubes for insertion instruments) not including mesh tubes are prone to elongation compared to conventional universal cords due to external stress and aging, adversely affecting tubes, such as channels, and signal lines inserted in through the cords, thus raising concerns about degraded reliability.

It is an objective of embodiments of the present disclosure to provide a bendable tube for an insertion instrument with ensured reliability, an insertion instrument with ensured reliability, and a method for manufacturing the bendable tube for the insertion instrument with ensured reliability.

### SUMMARY OF THE INVENTION

A bendable tube for an insertion instrument according to an embodiment of the present disclosure includes an elongated cylinder having a first end and a second end, and including a spiral tube formed by spirally winding a strip-shaped plate, and a tube having flexibility and provided on an outer circumferential surface of the spiral tube; two ferrules including a first ferrule fixed to the first end and a second ferrule fixed to the second end; and a wire having a third end and a fourth end, the third end being fixed to the first ferrule, the fourth end being fixed to the second ferrule, the wire being inserted in through the cylinder and having an elongation rate smaller than an elongation rate of the cylinder.

An insertion instrument according to an embodiment of the present disclosure is an insertion instrument to be inserted into a subject, and includes a bendable tube. The bendable tube includes an elongated cylinder having a first end and a second end, and including a spiral tube formed by spirally winding a strip-shaped plate, and a tube having flexibility and provided on an outer circumferential surface of the spiral tube; two ferrules including a first ferrule fixed to the first end, and a second ferrule fixed to the second end; and a wire having a third end and a fourth end, the third end being fixed to the first ferrule, the fourth end being fixed to the second ferrule, the wire being inserted in through the cylinder, and having an elongation rate smaller than an elongation rate of the cylinder.

A method for manufacturing a bendable tube for an insertion instrument according to an embodiment of the present disclosure includes producing a spiral tube by spirally winding a strip-shaped metal plate in a longitudinal axis direction; producing a cylinder by providing, on a surface layer side of the spiral tube, a tube having flexibility and extending in the longitudinal axis direction; attaching a ferrule to each of both ends of the cylinder; and inserting a wire in through the cylinder, the wire having an elongation rate smaller than an elongation rate of the cylinder, and fixing both ends of the wire to respective ones of the ferrules.

According to embodiments of the present disclosure, a bendable tube for an insertion instrument with ensured reliability, an insertion instrument with ensured reliability, and a method for manufacturing the bendable tube for the insertion instrument with ensured reliability can be provided.

### BRIEF DESCRIPTION OF THE DRAWINGS

Fig. 1 is a perspective view of an endoscope of an embodiment.
Fig. 2 is a perspective view of a universal cord of the embodiment.
Fig. 3 is a cross-sectional view along line III-III of Fig. 2.
Fig. 4 is an exploded perspective view of the universal cord of the embodiment.
Fig. 5 is a partial cross-sectional view of a universal cord of a modification example of the embodiment.
Fig. 6 is a flowchart of a method for manufacturing the universal cord of the embodiment.
Fig. 7 is a view of a core and a mold for manufacturing a tube of a reference example.
Fig. 8A is a cross-sectional view for describing a method for manufacturing the tube of the reference example.
Fig. 8B is a cross-sectional view for describing the method for manufacturing the tube of the reference example.
Fig. 8C is a cross-sectional view for describing the method for manufacturing the tube of the reference example.
Fig. 8D is a cross-sectional view for describing the method for manufacturing the tube of the reference example.

### DETAILED DESCRIPTION

### <Embodiments>

The following describes a universal cord for an endoscope, which is a bendable tube for an insertion instrument, using the drawings. The drawings based on the embodiments are schematic. The relationship between the thickness and width of each part and the ratio of the thickness of each part in the drawings differ from the actual ones. The drawings also contain parts in which dimensional relationships and ratios are different from each other. Illustration of some components and their reference numerals are omitted.

### <Endoscope Configuration>

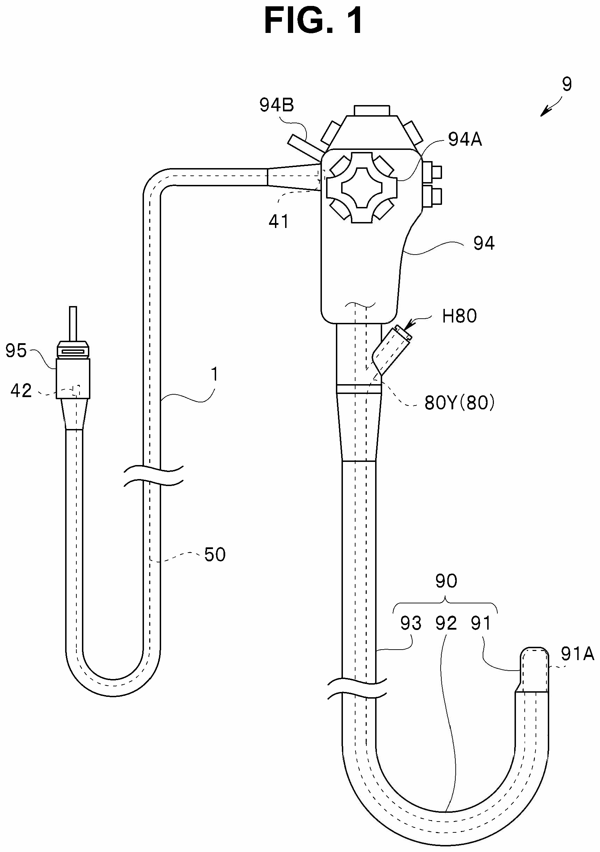

An endoscope 9 of an embodiment shown in Fig. 1 includes an insertion portion 90 to be inserted into a body of a subject, an operation portion 94 provided on a proximal end side of the insertion portion 90, and a universal cord 1 extending from the operation portion 94. In the universal cord 1, a plurality of tubes, such as a channel (suction conduit) 80, and a plurality of signal lines are inserted through.

The endoscope 9 is, as one example, a single-use endoscope to be discarded after a single use. The application of the endoscope 9 may be either medical or industrial.

The insertion portion 90 includes a distal end portion 91 on a distal end side, a bending portion 92 disposed on a proximal end side of the distal end portion 91, and an elongated flexible tube 93 connecting a proximal end side of the bending portion 92 to the operation portion 94, which are provided in a linked manner. Although not shown, an image pickup unit, an illumination optical system, and the like are disposed in a rigid member 91A of the distal end portion 91. The rigid member 91A is made of metal (for example, stainless steel) or resin (for example, epoxy resin, ABS resin).

It should be noted that connection sections of the respective members of the insertion portion 90 (between the distal end portion 91 and the bending portion 92, between the bending portion 92 and the flexible tube 93, and between the flexible tube 93 and the operation portion 94) are fixed by thread wrapping and further reinforced by resin. The connection sections may be fixed by pins instead of thread wrapping, or fixed only with resin. The connection sections of the respective members of the insertion portion 90 (between the distal end portion 91 and the bending portion 92, between the bending portion 92 and the flexible tube 93, and between the flexible tube 93 and the operation portion 94) are fixed by pins, screws, rivets, or the like, and further reinforced with resin such as adhesive. The connection sections may be fixed by only resin, or only pins, screws, rivets, or the like.

The operation portion 94 is provided with an insertion port H80 of a treatment instrument channel for introducing a treatment instrument or the like into the insertion portion 90. As will be mentioned later, the channel 80 serves not only as the treatment instrument channel but also as a suction tube, and therefore includes a Y-shaped branch portion 80Y.

On the operation portion 94, various operating switches, including a bending operation dial 94A, are provided. To the bending operation dial 94A, a plurality of bending operation wires (not shown) that are inserted through the flexible tube 93, with respective ends of the wires fixed to the bending portion 92, are fixed. The bending portion 92 bends in response to a rotational motion of the bending operation dial 94A.

Although not shown, on an outer circumferential surface of the ferrule of the flexible tube 93, a plurality of elongated protrusions is formed to engage with a plurality of V-grooves on an inner surface of a fixing hole of the operation portion 94. For this reason, the rotational direction of the bending operation dial 94A and the bending direction of the bending portion 92 can be easily aligned during manufacturing. It should be noted that by forming the V-grooves in a greater number than the number of the protrusions, it is possible to finely adjust the rotational direction and the bending direction. For example, if a plurality of V-grooves is formed at a 5-degree pitch along the circumferential direction, the rotational direction and the bending direction can be adjusted with an accuracy within 5 degrees to fix the flexible tube 93 to the operation portion 94.

In an endoscope in which the rigid member 91A includes a raising base (forceps elevator) for operating the protrusion direction of the treatment instrument, a raising base operation wire (not shown) for operating the raising base is inserted through the insertion portion 90 and is extendedly provided to an operation lever 94B of the operation portion 94. Rotational operation of the operation lever 94B is, for example, converted into linear motion by a moving member clamped between two rails, and the raising base operation wire is connected to a rod connected to the moving member. The rod, the bending operation wire, and the raising base operation wire are coated on outer surfaces thereof with a lubricating liquid (for example, silicone oil) to improve sliding properties relative to a bearing or the like.

The various wires inserted through the insertion portion 90 and the universal cord 1 may be inserted into a resin tube. As the resin tube, a block copolymer configured of a rigid polyamide block and a flexible polyether block may be preferred instead of expensive fluororesin. Block copolymers are relatively inexpensive and, by controlling the composition and ratio of a plurality of blocks, can achieve a desired flexibility and elasticity without requiring plasticizers.

### <Configuration of Universal Cord>

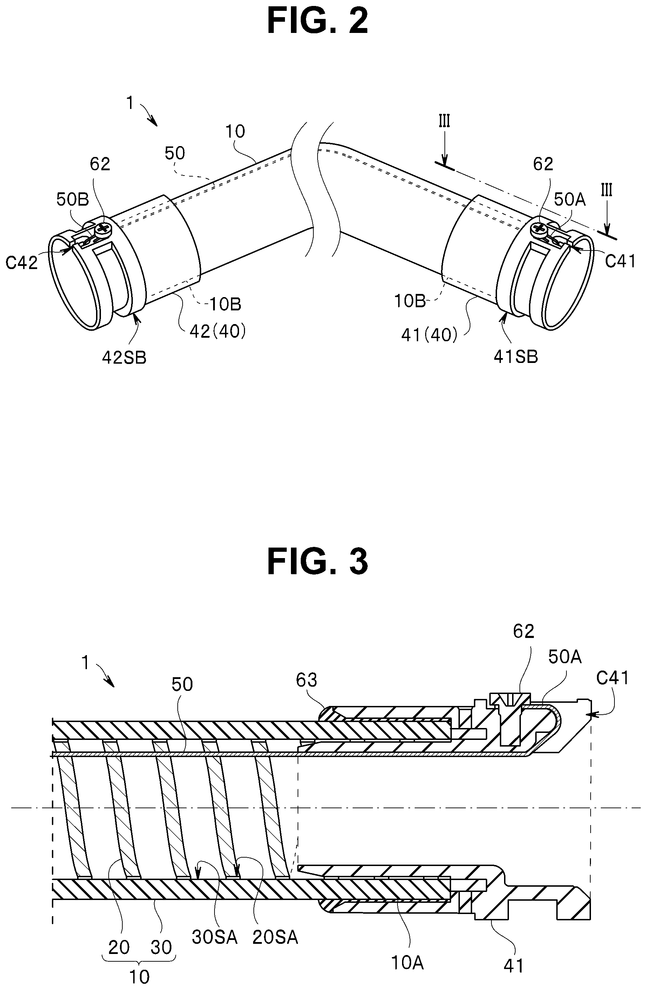

The universal cord 1 of the embodiment shown in Figs. 2 to 4 includes a cylinder 10, two ferrules 40 made of resin, and a wire 50. The cylinder 10 includes a spiral tube 20 formed by spirally winding a strip-shaped plate, and a tube 30 made of a flexible resin provided on an outer surface of the spiral tube 20.

The elongated cylindrical cylinder 10 includes a first end 10A and a second end 10B on an opposite side of the first end 10A. Of the two ferrules 40 having a substantially cylindrical shape, a first ferrule 41 is fixed to the first end 10A of the cylinder 10, and the second ferrule 42 is fixed to the second end 10B of the cylinder 10.

The wire 50 includes a third end 50A and a fourth end 50B on an opposite side of the third end 50A. The wire 50 has the third end 50A fixed to the first ferrule 41 and has the fourth end 50B fixed to the second ferrule 42. The wire 50 is inserted in through the cylinder 10, and has an elongation rate ΔL50 smaller than an elongation rate ΔL10 of the cylinder 10.

In the present specification, elongation rate ΔL is calculated from a length L1 at room temperature (25°C) under no load and a length L2 when one end is fixed and a load of 40 N is applied to the other end. That is, elongation rate ΔL% is ((L2 - L1) / L1) * 100.

Unlike a conventional universal cord, the universal cord 1 does not include a braided tube which is a mesh tube woven from fine metal wires, synthetic fibers, or the like.

The universal cord 1 is less expensive and more excellent in flexibility than a conventional universal cord including a mesh tube. The universal cord 1 has lower torsional rigidity, and therefore provides better operability than a conventional universal cord. Furthermore, the universal cord 1 eliminates the need for angle adjustment of the ferrules at both ends, decreasing the number of assembly steps and making manufacturing easier.

However, universal cords not including mesh tubes are prone to elongation compared to conventional universal cords due to external stress and aging, adversely affecting tubes, such as channels, and signal lines inserted in through the cords, thus raising concerns about degraded reliability.

However, the universal cord 1 includes the wire 50 to restrict elongation deformation of the cylinder 10, ensuring flexibility, kink resistance, and anti-elongation property, thereby ensuring reliability.

The following describes a detailed configuration of the universal cord 1.

The spiral tube 20 may be configured of a rigid resin plate having elasticity, not from a metal plate. The tube 30 is made of a flexible resin, such as polyurethane. The wire 50 may preferably be a cable formed by twisting together a plurality of metal wires made of, for example, stainless steel, as this yields excellent flexibility. The wire 50 may have an outer circumference covered by a resin film, or may be inserted into a resin tube having an inner diameter substantially the same as an outer diameter of the wire 50.

The elongation rate ΔL50 of the wire 50 may preferably be less than 10% of the elongation rate ΔL10 of the cylinder 10. If the elongation rate ΔL50 is less than 10% of the elongation rate ΔL10, the elongation deformation of the cylinder 10 can be restricted, making the universal cord 1 highly reliable.

For example, the elongation rate ΔL10 of the cylinder 10 is 0.7%, and the elongation rate ΔL50 of the wire 50 is 0.03%.

As shown in Fig. 2, the first ferrule 41 includes a cutout C41 which is a first through passageway through which the wire 50 is inserted. The second ferrule 42 includes a cutout C42 which is a second through passageway through which the wire 50 is inserted.

The wire 50 has the third end 50A fixed to an outer surface 41SB of the first ferrule 41, passes in through the cylinder 10 via the cutout C41, and via the cutout C42, has the fourth end 50B fixed to the outer surface 42SB of the second ferrule 42.

The wire 50 has the third end 50A fixed to the first ferrule 41, extends on the outer surface 41SB to an opposite side of the direction in which the second ferrule 42 is provided, and via the cutout C41, extends within the cylinder 10 in the direction where the second ferrule 42 is provided. The wire 50 has the fourth end 50B fixed to the second ferrule 42, extends on the outer surface 42 SB to an opposite side of the direction in which the first ferrule 41 is provided, and via the cutout C42, extends within the cylinder 10 in the direction in which the first ferrule 41 is provided. The wire 50 inserted in through the cylinder 10 may be fixed to at least one location on the inner surface of the cylinder 10.

The through passageway may be a through hole, not a cutout. However, a cutout facilitates the work of inserting the wire 50 more than a through hole.

The wire 50 is fixed to the first ferrule 41 and the second ferrule 42 by respective screws 62.

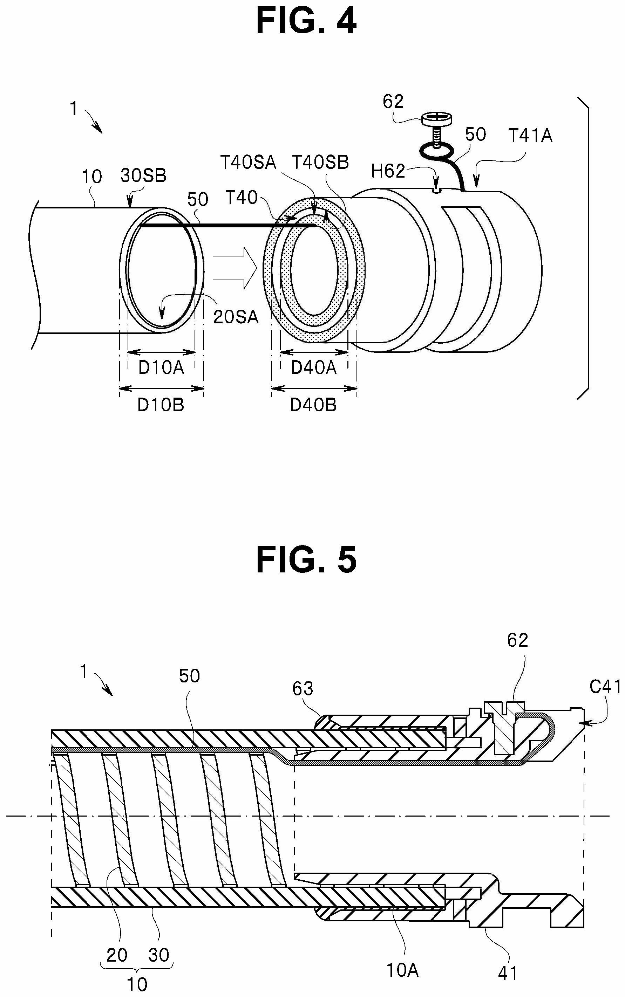

As with the first ferrule 41 shown in Figs. 4 and 5, although not shown, the second ferrule 42 also includes an annular groove T40 into which an end portion of the cylinder 10 is to be inserted. The groove T40 includes an inner wall surface T40SA having a diameter D40A slightly larger than an inner diameter D10A of the cylinder 10, and an outer wall surface T40SB having a diameter D40B larger than an outer diameter D10B of the cylinder 10.

When the cylinder 10 is inserted into the groove T40, the cylinder 10 expands in diameter by being pressed into the groove T40, such that the inner diameter D10A becomes the diameter of the inner wall surface of the groove T40. Since an end portion of the spiral tube 20 made of a metal plate is inserted into the groove T40 of the ferrule 40, there is no risk that the tube and wiring that are inserted in through the cylinder 10 are damaged by an end face of the metal plate.

Since each of the two ferrules 40 is made of a transparent material, for example, polycarbonate resin, the position of the distal end of the cylinder 10 inserted into the groove T40 is visible from the exterior. For this reason, it can be confirmed that the distal end of the cylinder 10 is inserted into a predetermined position in the groove T40. Each of the two ferrules 40 need only be partially configured of a transparent material as long as the distal end of the inserted cylinder 10 is visible from the exterior.

The space between the outer diameter D10B of the cylinder 10 and the outer wall surface T40SB of the groove T40 is filled with an adhesive 63. The cylinder 10 is firmly fixed to the ferrule because of being fixed by the adhesive 63 in a state of being inserted into the groove T40 of the ferrule 40.

That is, the cylinder 10, whose distal end portion is coated by the adhesive 63, is inserted into the groove T40, and the adhesive 63 undergoes a curing process. The adhesive 63 is, for example, a UV-curable resin capable of undergoing a curing process in a short time. The spiral tube 20 and the tube 30 have their respective end portions fixed to the ferrule 40 simultaneously. The adhesive fixing method is simpler and less expensive than, for example, fixing methods by brazing, pins, or the like.

The ferrule 40 may be joined to the cylinder 10 by so-called crimping. That is, the end portion of the cylinder 10 is inserted into the groove T40 of the ferrule 40, and then a force is applied to the outer circumference of the ferrule 40 to plastically deform the ferrule 40, thereby firmly fixing the ferrule 40 to the cylinder 10. A combination of crimp joining and adhesive may be used to fix the ferrule 40 to the cylinder 10.

The universal cord 1 has the first ferrule 41 fixed to the operation portion 94 of the endoscope 9, and the second ferrule 42 fixed to a connector 95 to be connected to an external instrument.

### <Method for Manufacturing Universal Cord>

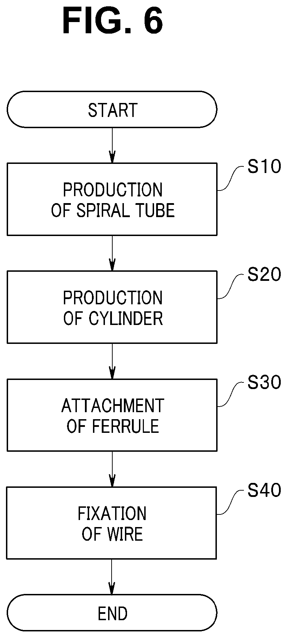

A method for manufacturing a universal cord will be described along the flowchart of Fig. 6.

### <Step S10>

The spiral tube 20 that is elongate is produced by spirally winding a strip-shaped metal plate in the longitudinal axis direction.

### <Step S20>

The spiral tube 20 is inserted in through the tube 30 having flexibility to produce the cylinder 10. As a specific method, the cylinder 10 is produced by, for example, providing the tube 30 having flexibility on a surface layer side of the spiral tube 20. For example, air is blown into the tube 30, and the spiral tube 20 is inserted into the tube 30 in a state of being expanded in diameter. The inner diameter of the tube 30 may preferably be slightly larger than the outer diameter of the spiral tube 20.

### <Step S30>

The ferrule 40 is attached to each of both ends of the cylinder 10. That is, the end of the cylinder 10, coated with the adhesive 63, is pressed into the groove T40 of the ferrule 40, followed by curing of the adhesive 63. The adhesive 63 is, for example, a UV-curable urethane acrylate resin. The curing is carried out by an ultraviolet irradiation method or by a combination of ultraviolet irradiation and heat curing.

### <Step S40>

The wire 50 having an elongation rate smaller than an elongation rate of the cylinder 10 is inserted in through the cylinder 10, and the third end 50A and the fourth end 50B of the wire 50 are fixed to the respective ferrules 40. That is, the screw 62 around which an end of the wire 50 is wound is inserted into a screw hole of the ferrule 40. It should be noted that the wire 50 may be fixed by a fastener including a screw or a pin, or by adhesive.

The wire 50 is set to a length to remain in a substantially straight state within the cylinder 10 when the cylinder 10 is placed in a straight state. In other words, the length of the wire 50 is substantially the same as the length of the cylinder 10.

According to the method for manufacturing the universal cord of the present embodiment, an inexpensive universal cord can be easily manufactured.

It should be noted that, as shown in Fig. 3, in the universal cord 1, the wire 50 is inserted in through an inner surface 20SA of the spiral tube 20. To prevent movement within the spiral tube 20, the wire 50 may be fixed to a guide member (not shown) provided to the spiral tube 20. As already described, since the inner diameter of tube 30 is slightly larger than the outer diameter of the spiral tube 20, the wire 50 may be inserted between the outer surface 20SB of the spiral tube 20 and an inner surface 30SA of the tube 30, as shown in Fig. 5.

### <Reference Example>

As already described, the channel 80 (suction tube) of the endoscope 9 includes the branch portion 80Y. The endoscope 9 is provided with another channel conduit, for example, a gas/liquid supply tube, not shown. In the gas/liquid supply tube, there also exists a location where a plurality of tubes is connected into one, similar to the branch portion 80Y. When the location (branch portion) where a plurality of tubes is connected into one is configured using a Y-shaped tube made of metal, the cost increases. Even when the location is configured using a Y-shaped tube made of resin, the assembly cost remains high, and the location becomes a waste when the endoscope is subject to disposal. For this reason, there is a need to reduce the cost by not using a Y-shaped tube.

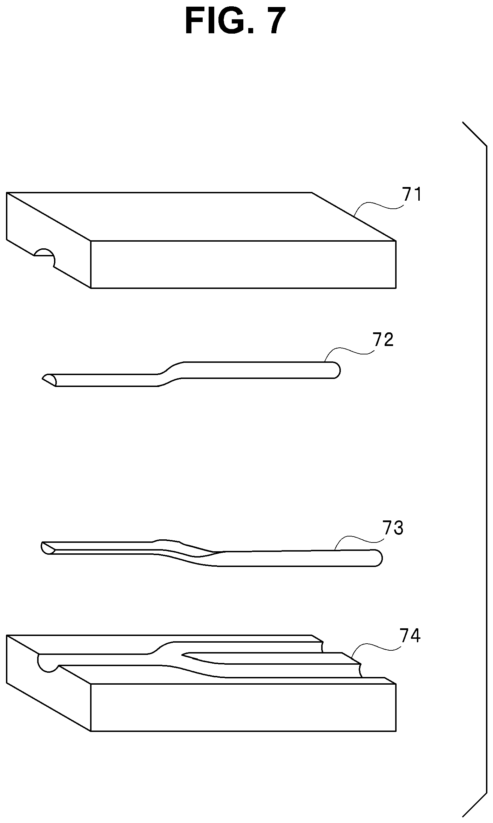

To manufacture a channel 81, such as the gas/liquid supply tube including a branch portion, an upper mold 71, an upper core 72, a lower core 73, and a lower mold 74 are used, as shown in Fig. 7. An outer circumferential surface of a set of cores, which is a combination of the upper core 72 and the lower core 73, has the same shape as an inner peripheral surface of a hollow portion of the channel 81. The upper core 72 and the lower core 73 each have a circular cross-section on one end side and a semicircular cross-section at the other end side. The upper core 72 and the lower core 73 are combined with their flat surfaces facing each other at the semicircular cross-section parts, and are used as a set of cores.

An inner peripheral surface of a set of molds, which is a combination of the upper mold 71 and the lower mold 74, has the same shape as an outer circumferential surface of the hollow portion of the channel 81.

### <Method for Manufacturing Channel>

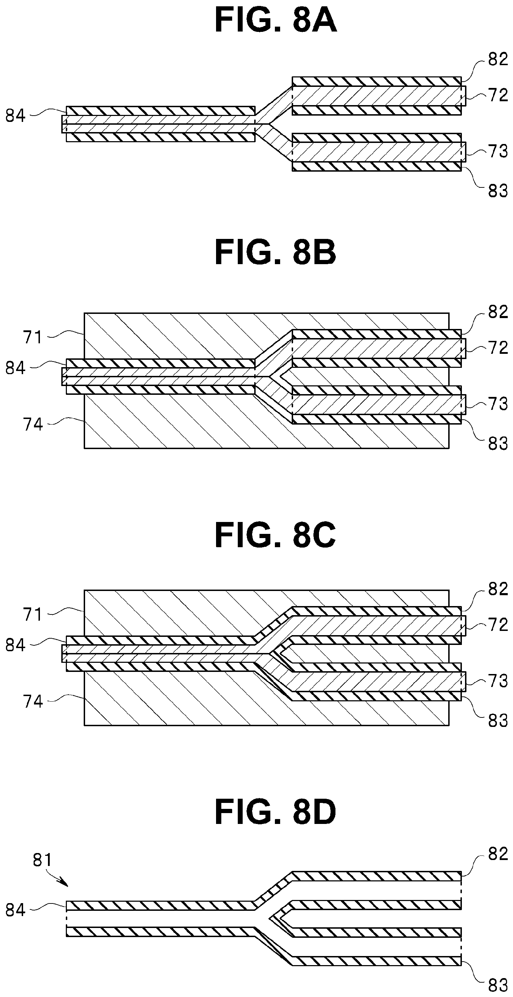

A method for manufacturing a channel will be described using Figs. 8A to 8D. Figs. 8A to 8D are cross-sectional views along a longitudinal direction of the channel.

As shown in Fig. 8A, a columnar part of the upper core 72 is inserted into a first resin tube 82, and a columnar part of the lower core 73 is inserted into a second resin tube 83. A columnar part formed by combining the upper core 72 and the lower core 73 is inserted into a third resin tube 84.

The first resin tube 82, the second resin tube 83, and the third resin tube 84 are made of a resin that melts when heated.

As shown in Fig. 8B, a set of cores, into which the first resin tube 82, the second resin tube 83, and the third resin tube 84 are inserted, is placed in the space between the upper mold 71 and the lower mold 74.

As shown in Fig. 8C, when a part where the first resin tube 82 and the second resin tube 83 face the third resin tube 84 is heated, the facing part melts to fuse the three tubes together.

As shown in Fig. 8D, after the upper mold 71 and the lower mold 74 are removed, the upper core 72 and the lower core 73 are removed, thereby completing the channel 81 including the branch portion 80Y.

As described above, the method for manufacturing the channel 81, such as the gas/liquid supply tube of the endoscope 9, includes arranging in parallel the first resin tube 82 which supplies a first fluid to the operation portion 94 from the exterior, and the second resin tube 83 into which the treatment instrument is to be inserted from the operation portion 94. The third resin tube 84, one end of which is placed in the distal end portion 91 of the insertion portion 90 to be inserted into the subject, and the other end of which is placed in the operation portion 94, is caused to face the first resin tube 82 and the second resin tube 83. The upper core 72 is inserted through the first resin tube 82 and the third resin tube 84, and the lower core 73 to be combined with the upper core 72 is inserted through the second resin tube 83 and the third resin tube 84. The first resin tube 82, the second resin tube 83, and the third resin tube 84, through all of which the upper core 72 and the lower core 73 are inserted, are mounted between the upper mold 71 and the lower mold 74. The part where the first resin tube 82 and the second resin tube 83 face the third resin tube 84 is heated between the upper mold 71 and the lower mold 74. By fusing the part where the three tubes face each other, the channel 81 wherein the first resin tube 82, the second resin tube 83, and the third resin tube 84 communicate via the branch portion is produced.

It should be noted that, while the above-mentioned method for manufacturing the channel 81 is intended for use in a branch part of a gas/liquid supply tube, a similar manufacturing method may also be employed for portion 80Y, which is the branch portion of the channel 80 including a suction tube and the like.

The above description used a universal cord of an endoscope as an example of a bendable tube for an insertion instrument. However, the bendable tube may also be a catheter or an insertion portion of an endoscope.

The present disclosure is not limited to the above-mentioned embodiments, etc., and various modifications, combinations, and applications are possible without departing from the gist of the disclosure.

The present application claims the benefit of priority based on U.S. Provisional Application No. 63/621,631, filed in the United States on January 17, 2024, and the disclosure therein is incorporated herein by reference to the specification, claims, and drawings of the present application.

### DESCRIPTION OF THE REFERENCE NUMERALS

- 1: universal cord
- 9: endoscope
- 10: cylinder
- 20: spiral tube
- 20SA: inner surface
- 20SB: outer surface
- 30: tube
- 30SA: inner surface
- 40: ferrule
- 41: first ferrule
- 41SB: outer surface
- 42: second ferrule
- 42SB: outer surface
- 50: wire
- 63: adhesive
- 71: upper mold
- 72: upper core
- 73: lower core
- 74: lower mold
- 80, 81: channel
- 82: first resin tube
- 83: second resin tube
- 84: third resin tube
- 90: insertion portion
- 91: distal end portion
- 92: bending portion
- 93: flexible tube
- 94: operation portion

## Claims

1. A bendable tube for an insertion instrument, comprising:
an elongated cylinder having a first end and a second end, and including a spiral tube formed by spirally winding a strip-shaped plate, and a tube having flexibility and provided on an outer circumferential surface of the spiral tube;
two ferrules including a first ferrule fixed to the first end and a second ferrule fixed to the second end; and
a wire having a third end and a fourth end, the third end being fixed to the first ferrule, the fourth end being fixed to the second ferrule, the wire being inserted in through the cylinder and having an elongation rate smaller than an elongation rate of the cylinder.

2. The bendable tube for the insertion instrument according to claim 1, wherein the elongation rate of the wire is less than 10% of the elongation rate of the cylinder.

3. The bendable tube for the insertion instrument according to claim 1, wherein the wire is inserted in through an inner surface of the spiral tube.

4. The bendable tube for the insertion instrument according to claim 1, wherein the wire is inserted between an outer surface of the spiral tube and an inner surface of the tube.

5. The bendable tube for the insertion instrument according to claim 1, wherein the first ferrule includes a first through passageway through which the wire is to be inserted, and the second ferrule includes a second through passageway through which the wire is to be inserted.

6. The bendable tube for the insertion instrument according to claim 5, wherein the wire has the third end fixed to an outer surface of the first ferrule, is inserted through the first through passageway, passes through the cylinder, is inserted through the second through passageway, and has the fourth end fixed to an outer surface of the second ferrule.

7. The bendable tube for the insertion instrument according to claim 1, wherein the wire is fixed to each of the two ferrules by a fastener including a screw or a pin.

8. The bendable tube for the insertion instrument according to claim 1, wherein the wire has the third end fixed to the first ferrule, extends to an opposite side of a direction in which the second ferrule is provided, and extends in the direction in which the second ferrule is provided.

9. The bendable tube for the insertion instrument according to claim 1, wherein the wire has the fourth end fixed to the second ferrule, extends to an opposite side of a direction in which the first ferrule is provided, and extends in the direction in which the first ferrule is provided.

10. The bendable tube for the insertion instrument according to claim 1, wherein the two ferrules include an annular groove into which the first end or the second end of the cylinder is to be inserted.

11. The bendable tube for the insertion instrument according to claim 10, wherein the groove includes an inner wall surface having a diameter larger than an inner diameter of the cylinder and an outer wall surface having a diameter larger than an outer diameter of the cylinder.

12. The bendable tube for the insertion instrument according to claim 11, wherein each of the two ferrules allows the cylinder inserted into the groove to be visible from an exterior.

13. The bendable tube for the insertion instrument according to claim 12, wherein each of the two ferrules is at least partially configured of a transparent material.

14. The bendable tube for the insertion instrument according to claim 11, wherein the cylinder is fixed in the groove by an adhesive.

15. The bendable tube for the insertion instrument according to claim 1, wherein the wire includes a cable formed by twisting together a plurality of metal wires.

16. The bendable tube for the insertion instrument according to claim 1, wherein an insertion instrument is a single-use endoscope to be disposed of after a single use.

17. The bendable tube for the insertion instrument according to claim 16, wherein
the bendable tube is a universal cord of the endoscope, and
the bendable tube does not include a braided tube, the braided tube being a mesh tube that covers the spiral tube and being woven from fine metal wires or synthetic fibers.

18. The bendable tube for the insertion instrument according to claim 17, wherein the first ferrule is fixed to an operation portion of the endoscope, and the second ferrule is fixed to a connector configured to be connected to an external instrument.

19. An insertion instrument comprising a bendable tube configured to be inserted into a subject,
the bendable tube including:
an elongated cylinder having a first end and a second end, and including a spiral tube formed by spirally winding a strip-shaped plate, and a tube having flexibility and provided on an outer circumferential surface of the spiral tube;
two ferrules including a first ferrule fixed to the first end, and a second ferrule fixed to the second end; and
a wire having a third end and a fourth end, the third end being fixed to the first ferrule, the fourth end being fixed to the second ferrule, the wire being inserted in through the cylinder and having an elongation rate smaller than an elongation rate of the cylinder.

20. A method for manufacturing a bendable tube for an insertion instrument, the method comprising:
producing a spiral tube by spirally winding a strip-shaped metal plate in a longitudinal axis direction;
producing a cylinder by providing, on a surface layer side of the spiral tube, a tube having flexibility and extending in the longitudinal axis direction;
attaching a ferrule to each of both ends of the cylinder; and
inserting a wire in through the cylinder, the wire having an elongation rate smaller than an elongation rate of the cylinder, and fixing both ends of the wire to respective ones of the ferrules.
